# EUROPEAN PATENT APPLICATION

(11) **EP 3 539 445 A1**
(43) Date of publication of application: **18.09.2019**
(21) Application number: 18161746.5
(22) Date of filing: 14.03.2018
(51) Int. Cl.: A61B 1/00, A61B 1/07, G02B 23/24

(54) **METHOD FOR MANUFACTURING A TIP HOUSING**

(71) Applicant: Ambu A/S, 2750 Ballerup (DK)
(72) Inventor: VILHELMSEN, Erik Øllgaard, 3060 Espergærde (DK); SØRENSEN, Morten, 2750 Ballerup (DK)
(74) Representative: AWA Denmark A/S

(57) **Abstract**

Method for manufacturing a tip housing (5) for the distal tip of a vision device such as an endoscope. The method comprises providing a moulding tool, introducing in the moulding tool a first housing material, introducing in the moulding at least one further housing material different from the first housing material, allowing the at least one further housing material to set and form a combined housing component with the first moulding material, removing the combined housing component from the moulding tool.

## Description

The present invention relates to vision devices such as but not limited to endoscopes, more specifically to a housing for the tip of the vision device and the manufacture thereof.

Vision devices, such endotracheal tubes and endoscopes are well known devices for visually inspecting inaccessible places such as human body cavities. Typically, the endoscope comprises an elongated insertion tube with a handle at the proximal end as seen from the operator and visual inspections means, such as a built-in camera, at the distal end of the elongated insertion tube. Electrical wiring for the camera and other electronics such as LED lighting accommodated in the tip part at the distal end run along the inside of the elongated insertion tube from the handle to the tip part. Instead of using cameras, endoscopes may also be fibre-optic, in which case the optical fibres run along inside of the elongated insertion tube to the tip part.

In order to be able to manoeuvre the endoscope inside the body cavity, the distal end of the endoscope may comprise a bending section with increased flexibility, e.g. a number of articulated segments of which the tip part forms the distalmost segment. This is typically done by tensioning or slacking pull wires also running along the inside of the elongated insertion tube from the tip part through the remainder of articulated segments to a control mechanism of the handle. Furthermore, a working channel may run along the inside of the insertion tube from the handle to the tip part, e.g. allowing liquid to be removed from the body cavity or allowing the insertion of surgical instruments or the like into the body cavity.

As the name indicates, endoscopes, are used for seeing inside things, such as lungs or other human body cavities of a patient. Modern endoscopes are therefore typically equipped with a least one camera or similar image capturing device at the distal tip of the endoscope. Provided that sufficient light is present, this allows the operator to see where the endoscope is steered and to set the target of interest once the tip has been advanced thereto. This therefore normally requires illumination of the area in front of the distal tip of the endoscope, in particular the field of vision of the camera(s). One known way of achieving such illumination is to provide the above mentioned LED lighting using one or more Light Emitting Diodes (LEDs) in the tip of the endoscope, as e.g. mentioned in WO2014/106511 disclosing a disposable endoscope.

When, as in the present invention, the insertion tube of the endoscope is intended to be inserted into a human body cavity, the insertion tube needs to be sealed in a watertight manner. This is in particular the case for the distal tip part because it accommodates the camera, LED(s) and other delicate electronics, prone to malfunction or destruction if exposed to humidity.

One known way of sealing the tip part of an endoscope is disclosed in WO2010/066790. In this document a transparent monolithic housing is formed around the electronics and working channel by placing the electronics and the tube forming the working channel in a mould of transparent material, such as silicone. A transparent UV curable resin is then inserted from the bottom of the mould to avoid bubbles to form in the transparent resin. Because the resin rises slowly from the bottom, the air is slowly expelled from top of the mould, without any risk of air bubbles being trapped in the mould. The resin is then cured using UV irradiation through the transparent mould to form the monolithic housing. However, forming a monolithic housing in this way has some drawbacks. One is that it is a somewhat slow process. Another is that it can be difficult to position and maintain the components precisely in position during the insertion of the resin. Thus, the camera or LEDs may in few cases be off-set sideways or a thin transparent layer may inadvertently be created in front of the camera and/or LEDs, thereby reducing the imaging qualities of the tip part. This will lead to the product being discarded in the quality control, thereby increasing overall cost of manufacturing.

A further problem is the general transparency of the housing, which derives from that fact that the resin itself is and - needs to be, transparent in order for the UV radiation to penetrate and cure the resin. This may result in undesired stray light from the LEDs which through the transparent housing itself impinges on the sensor of the camera and may disturb the captured images.

Furthermore, the materials which have good optical properties in terms of refraction index and transparency may not have good adhesion properties for e.g. sealant material, glue used for attaching outer sheaths or similar that involves adhesion.

Based on this prior art it is a first object of the invention to provide a housing for a tip part of an endoscope and an endoscope with such a housing for the tip part which do not suffer from the above drawbacks.

According to a first aspect of the invention this object is achieved by a method for manufacturing a tip housing for a vision device, such as the distal tip of an endoscope, said method comprising providing a moulding tool, introducing in the moulding tool a first housing material, introducing in the moulding tool at least one further housing material different from the first housing material, allowing the at least one further housing material to set and form a combined housing component with the first moulding material, removing the combined housing component from the moulding tool.

This allows the provision of an integrated unit for the tip housing having different areas with different desired properties.

According to a first preferred embodiment, the moulding tool comprises a first cavity, a second cavity and a core. In particular, in injection moulding this is advantageous as the moulded object normally shrinks during cooling and therefore tends to stick to the core.

According to a preferred embodiment, the first housing material is allowed to set before the at least one further housing material is introduced. This provides well defined boundaries between the two materials in the final integrated unit. Moreover, it allows the first mould to stick to the core for the introduction into the second cavity of the moulding tool.

According to another preferred embodiment, the volume of the at least one further housing material introduced in the mould is smaller than the volume of the first housing material introduced in the mould. This is in particular of advantage when the second material is more brittle than the first material, because having a smaller volume thereof will make it less prone to stick to the mould due to shrinking, thereby making it easier to extract from the mould. Therefore, the second material may also be injected at higher pressure than the first material, because a high pressure used for the first material would make it more prone to sticking to the mould and/or cores, in turn making removal more difficult.

According to a further preferred embodiment, the at least one further housing material is selected from the group comprising thermoplastic materials, thermoset materials and elastomers. These materials render themselves for injection molding of at least the second housing material. Injection moulding is efficient in terms of quick reproduction of identical items.

Accordingly, according to an especially preferred embodiment, the introduction of the first and/or the further housing material forms part of an injection moulding process.

According to another preferred embodiment, the at least one further housing material comprises a transparent housing material. Injecting the transparent material as the second housing material in many cases is advantageous, because the transparent materials which are preferred for their optical properties, may then be introduced under higher pressure than the first material. This, in turn, reduces shrinking and better control of the optical properties of the final product is therefore obtained. Furthermore, because the more brittle material constitutes only a minority part of the total housing material, it is easier to remove from the mould. Accordingly, it is also preferred when the first housing material is opaque.

Albeit the first housing material could also be selected for other properties, such as good adhesion to sealant materials and adhesives. Thus, according to a further preferred embodiment, the set first material has better adhesion properties to glue than the second material.

According to another preferred embodiment, first cavity and the second cavity have generally cylindrical shapes. This results in a generally cylindrical tip housing which in turn is suitable for the endoscopes made with tip housing according to the present invention.

The invention will now be described in greater detail based on non-limiting exemplary embodiments and with reference to the drawings on which:
Fig. 1 shows an isometric view of a distal tip part of an endoscope with a housing part according to the present invention,
Fig. 2 shows a cross-section of the distal tip part of Fig. 1 taken along the line II-II,
Fig. 3 shows a cross-section corresponding to that of Fig. 3 of the housing part of the distal tip only,
Fig. 4 shows a first exploded view of the housing part of Fig. 1,
Fig. 5 shows a second exploded view of the housing part of Fig. 1,
Fig. 6 shows an isometric view of a first mould cavity for the use in manufacturing the housing part of figs. 1 to 5,
Fig. 7 shows an isometric view of a core for the use in manufacturing the housing part of figs. 1 to 5,
Fig. 8 shows a cross-section along the line VIII-VIII of fig. 6 with the core inserted,
Fig. 9 shows a cross section along the line IX-IX of fig. 7 with the core inserted,
Fig. 10 shows an isometric view of a second mould cavity for the use in manufacturing the housing part of figs. 1 to 5
Fig. 11 shows the core of Fig. 7 with a first housing part attached,
Fig. 12 shows a cross-section along the line XII-XII of fig. 10 with the core inserted,
Fig. 13 shows a cross section along the line XIII-XIII of fig. 10 with the core inserted, and
Fig. 14 shows an endoscope with a distal tip part in accordance with the invention.

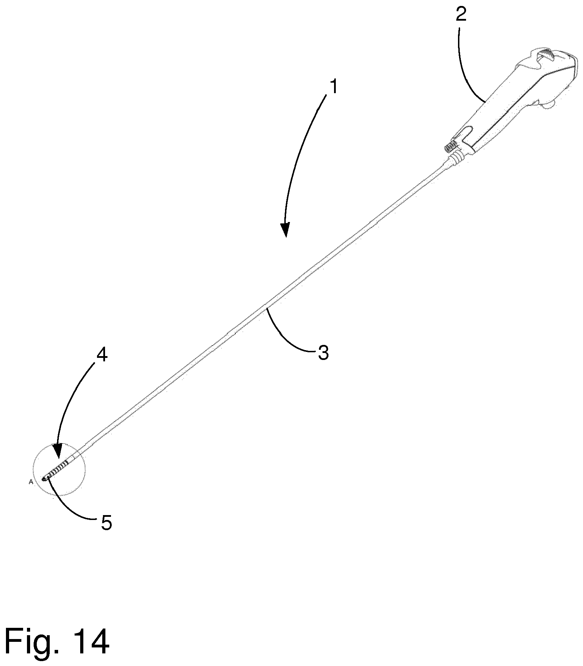

Turning first to Fig. 14, an endoscope 1 exemplifying the vision device according to the invention is shown. The endoscope 1 comprises a handle 2 at the proximal end, an insertion tube 3 extending towards the distal end where it comprises an articulated bending section 4, which as the most distal segment has a distal tip part 5 according to the invention. Though omitted for illustration purposes the articulated bending section 4 will normally be covered by a suitable sleeve, connected at least at its own distal end to the distal tip part 5, e.g. by means of an adhesive. This as such is conventional and e.g. known from the aforementioned WO2014/106511. The endoscope 1 of the present invention is intended as a disposable endoscope to be thrown away after use and low manufacturing costs is therefore an important issue.

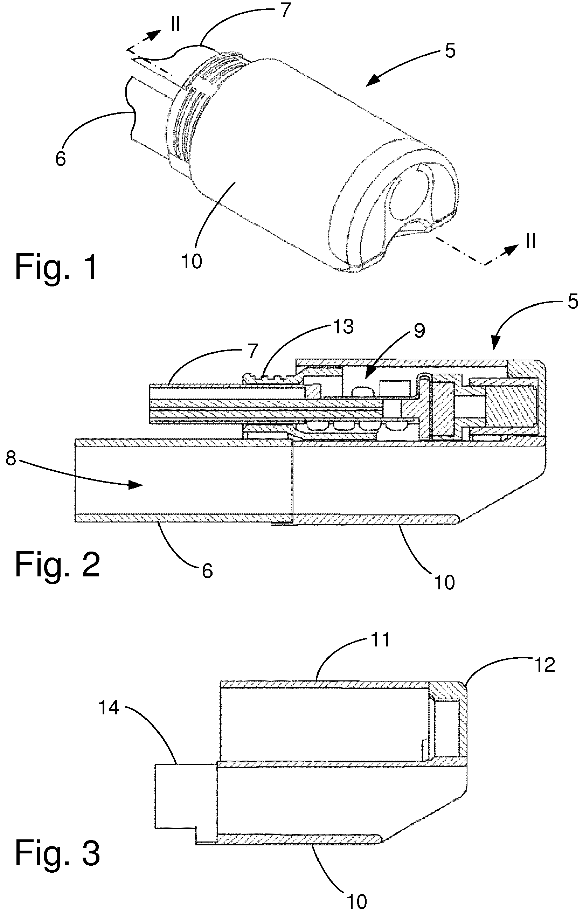

In Fig. 2 the distal tip part 5 can be seen in greater detail. Two tubular members 6, 7 leads to the tip part from the proximal handle 2. The first tubular member 6 provides a working channel 8 for the endoscope 1. The second tubular member 7 serves as a conduit for electrical cables and/or optical fibres and/or illumination fibres, depending on which kind of illumination and imaging the endoscope 1 relies on. In the illustrated embodiment, the imaging and illumination relies on an electronics section 9 with LEDs and a video camera accommodated in the distal tip part 5, but the invention is not limited thereto. Rather, the present invention relates to the tip housing 10 rather than the details of what is accommodated therein.

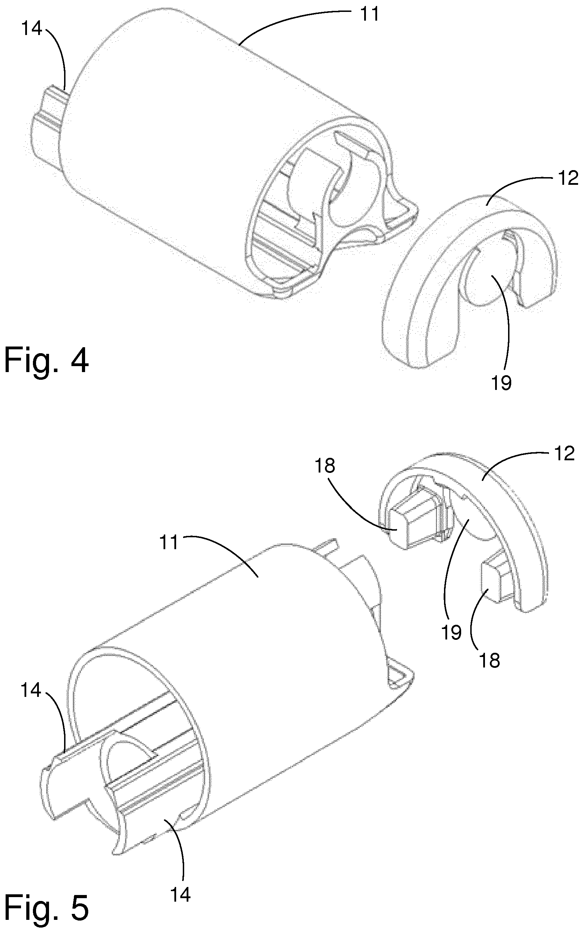

In Fig. 3 the tip housing 10 is shown on its own. As will be noticed from the different hatching as well as the exploded views of Fig. 4 and 5, the tip housing comprises two different materials 11, 12. To keep cost down, both of these materials are preferably polymer materials, in particular thermoplastic materials suitable for injection moulding, but also thermoset materials and/or elastomers can be used. The second material 12 is a transparent material allowing light from the light source(s) such as LED(s) to pass and illuminate objects beyond the distal end of the endoscope 1. Accordingly, the second material 12 should have good optical properties, e.g. in terms of transparency and high index of refraction. However, not all materials render themselves for economically feasible manufacture and the use in environments for which the endoscope is intended. The second material 12 is therefore preferably an injection mouldable polymer material such as polycarbonate, but other thermoplastic materials such as COP, COC and PMMA are also envisaged as are thermoset materials such as LSR (Liquid Silicone Rubber). One advantage of such softer and/or more elastic materials is their impact resistance. A softer and/or more elastic material would also allow for an increase in the transverse dimensions of the core 16 as the softer and/or more elastic material would yield during withdrawal thereof from the mould cavity 17 in the moulding process described below.

The first material 11 on the other hand need not have good optical properties and may therefore be selected based on entirely different criteria.

In particular the first material may be an opaque material. This will allow stray light from e.g. the light source to be absorbed and not disturb the images captured by the vision receptor, be it a video camera, an imaging chip, or an optical fibre. If opacity is the only desire, the first material 11 may essentially be the same as the second material 12, i.e. the same plastic material with a filler or a dye to make it opaque. This ensures that the first and second materials are very compatible, allowing them to bond well together and ensure a water and air tight housing part 5.

Also, the first material 11 may also be chosen for good adhesion properties to other materials, e.g. other parts of the endoscope 1. Such good adhesion properties could be good bonding to sealant materials, used for sealing the proximal end of the housing, e.g. around the lead-in 13 for electrical cables to the electronics section 9, to prevent ingress of water and other pollutants that could potentially harm the electronics section 9. Also, good bonding to an adhesive used for attaching the external sleeve around the bending section is advantageous.

Furthermore, the first material may be soft at least compared to the second material. In particular, it may be so soft that rather than attaching a sleeve to it, it may itself form a sleeve for the articulated bending section 4 to which it is attached as the most distal segment. Furthermore, this would facilitate or enable demoulding of undercut features inside the housing because the material may deform elastically.

Evidently, there are numerous other design choices that come into play for the selection of the most suitable material for the first material 11, provided of course that no further materials are used. The latter is, however, far from excluded by the present invention. Thus, if for the attachment of the tubular member 6 a number of spring like protrusions 14 are provided for holding the end of the tubular member 6, the material should be resilient enough to provide sufficient holding force, but of course with suitable adhesive properties, the tubular member could also be glued to the inside of the protrusions 14 or a similar receptacle.

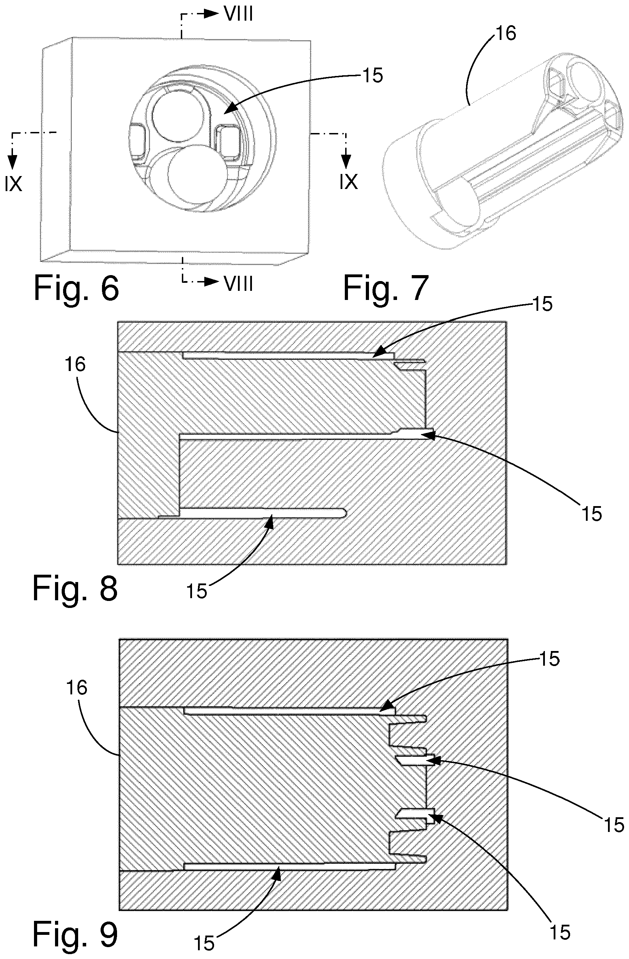

To achieve good tightness between the first material 11 and the second material 12 they are according to the invention moulded together to form one integrated unit. A moulding process for this will be described below with reference to Figs. 6 to 13.

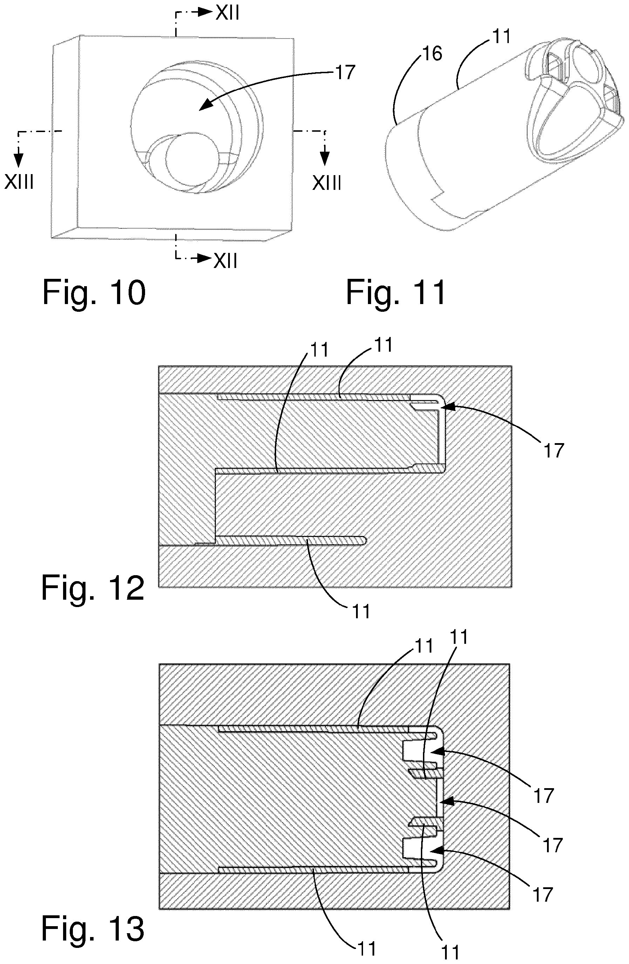

Turning first to Fig. 6, an isometric view of a generally cylindrical first mould cavity 15 forming part of moulding tool for the use in the moulding process is shown, albeit only schematically as inlets and the like have been omitted. The moulding tool further comprises a core 16 adapted to be inserted into the first mould cavity to form a first configuration of the moulding tool as illustrated in the cross-sections of Figs. 8 and 9. Assuming that the first material is a thermoplastic material, then in this first configuration the hot liquefied first material 11 is injected into the mould cavity 15 under a suitable pressure. When the liquefied first material 11 cools and solidifies it shrinks as most material do when they cool. Thus, when, after the cooling, the core 16 is removed from the first mould cavity 15 the solidified first material 11 will stick to the core 16 and be removed with it. This is in particular the case if additional first material 11 is not injected under sustained pressure during the cooling stage. Fig. 11 shows the core 16 with the first material 11 stuck to it. If the first material is a thermoset material it will instead be injected in a cold state and subsequently heated to set or vulcanize the material.

The core 16 with the first material stuck to it is then used as a what is effectively a new core in a second generally cylindrical mould cavity 17 forming as second configuration of the moulding tool, as can be seen in the cross-sections of Figs. 12 and 13. Here it will also be noted that in the preferred embodiment the volume of the resulting cavity 17 is smaller than the volume of the cavity 15. Accordingly, the volume of the second material introduced in the second moulding stage is smaller than the volume of the first material introduced in the first stage. Using a smaller volume results in a smaller part, in turn keeping the surface area with which it may adhere to the mould down. Filling the resulting cavity 15 at a higher pressure, and injecting additional second material as the second material cools, is therefore possible without the resulting housing sticking unnecessarily in the mould. The larger volume of the first material may be introduced at lower pressure ensuring that the shrinking material sticks to the core 16 rather than the mould, allowing for easy removal and reinsertion.

This remaining cavity can now be filled with the hot liquefied second material 12 which fuses with the first material 11 when it solidifies and forms an integral unit as the housing part 5. As described above in conjunction with the first material this may be a hot liquefied injection with subsequent cooling if the second material is a thermoplastic material or a cold injection with subsequent heating if the second material is a thermosetting material. Since this part formed from the second material 12 is located in front of the light source(s) and the vision receptor, be it a video camera, an imaging chip, or an optical fibre it is important to also ensure good optical properties of the finished part. Thus, the second material 12 is preferably injected under high pressure to ensure good filling of the cavity 17 without internal bubbles, and with sustained high pressure under the solidifying stage to avoid shrinking, which could deteriorate smoothness of the front surface and cause internal cavities. Furthermore, apart from the optical advantages gained form injecting the transparent second material 12 last, injecting this second material which will normally have a smaller volume is advantageous. This allows the above mentioned injection at higher pressures because the smaller volume decreases the risk of the shrinking material sticking to the core and/or moulds, even if additional material is injected to compensate for the shrinking.

After the second material 12 has finished solidifying, the housing part 5 comprising the bonded together first and second materials 11, 12 still sticks to the core 16 and may be removed from the second moulding cavity 17 with it, when the moulding tool is opened by retracting the core 16. The finished housing part 5 may then be separated from the core 16 in a conventional manner by means of ejectors in the core 16 or in any other suitable manner, e.g. using a robot.

The moulding tool may preferably comprise two identical cores for one set of first and second cavities. With a suitable alternation arrangement, e.g. a revolver, one core 16 may be used in the first moulding cavity while simultaneously the other core 16 with the solidified first material 11 thereon is used in the second moulding cavity and subsequently vice versa in an alternating manner. This arrangement may of course be multiplied with more sets of first and second cavities in sequence and a corresponding number of cores used in these cavities in one after the other.

The layout of the first and second moulding cavities 15, 17 and the core 16 may differ from the illustrated embodiment resulting in a different housing part 5. Thus, either or both of first material and second material 12 could extend longer in the lengthwise direction i.e. from distal end towards proximal end. The layout of an opaque material could be shaped specifically to the output of the light sources. Likewise, the layout of the transparent material could be shaped specifically to the light sources. As best seen in Fig. 5 this could involve the transparent second material 12 comprising integrated light guides 18, allowing the angular light distribution from LED light sources to be adapted to the field of view a video camera used as the image receptor. As can be seen the light guides in the present example are truncated pyramids with a generally rectangular cross-section which has been found useful for the rectangular image capture chip of a video camera.

Also, the protective window 19 in front of the image receptor could comprise curved surfaces in order to act as a lens.

Furthermore, even though the description above has been given for sequential moulding it is not excluded that the housing part may be moulded in a co-injection process where the first and second housing materials are introduced e.g. simultaneously into a single moulding cavity through different inlets. This will allow control to be maintained over the location of the different materials without having to open, modify and reclose the mould, i.e. moving the cores 16 with respect to the moulding cavities 15, 17.

With the housing part provided as an integral unit comprising two different materials it becomes possible to optimize the housing part for more than one parameter, e.g. for transparency vs. opacity, transparency vs. adhesion, brittleness vs. toughness, etc. The integral unit may be provided by other methods than the preferred one described above. In particular if injection moulding is used, an opposite arrangement could be used, i.e. a single moulding cavity, and two different cores inserted sequentially in that single moulding cavity, the first moulded part then remaining in the cavity when the second core is inserted. Analogous to the above, a number of identical cavities could then be used.

## Claims

1. Method for manufacturing a tip housing for the distal tip of a vision device, such as an endoscope, said method comprising
providing a moulding tool,
introducing in the moulding tool a first housing material,
introducing in the moulding at least one further housing material different from the first housing material,
allowing the at least one further housing material to set and form a combined housing component with the first moulding material,
removing the combined housing component from the moulding tool.

2. Method according to claim 1, wherein the moulding tool comprises a first mould cavity, a second mould cavity and a core.

3. Method according to any one of the preceding claims, wherein the first housing material is allowed to set before the at least one further housing material is introduced.

4. Method according to any one of the preceding claims, wherein the volume of the at least one further housing material introduced in the mould is smaller than the volume of the first housing material introduced in the mould.

5. Method according to any one of the preceding claims, wherein the at least one further housing material is selected from the group comprising thermoplastic materials, thermoset materials, and elastomers.

6. Method according to any one of the preceding claims, wherein the at least one further housing material comprises a transparent housing material.

7. Method according to any one of the preceding claims, wherein the first housing material is opaque.

8. A method according to any one of the preceding claims wherein the set first material has better adhesion properties to glue than the set second material.

9. Method according to any one of the preceding claims, wherein the first cavity and the second cavity have generally cylindrical shapes.

10. Method according to any one of the preceding claims wherein the introduction of the first and/or the further housing material forms part of an injection moulding process.

11. A tip housing for the distal tip of a vision device such as an endoscope obtained by the process according to any one of the preceding claims.

12. A tip housing according to claim 11, wherein the at least one further housing material is a thermoplastic material.

13. A tip housing according to claim 11 or 12, wherein the at least one further housing material comprises a transparent housing material.

14. A tip housing according to any one of claims 11 to 13, wherein the first housing material is opaque.

15. A tip housing according to any one of claims 11 to 14, wherein the set first material has better adhesion properties to glue than the set second material.

16. A tip housing according to any one of claims 11 to 15, wherein the first cavity and the second cavity have generally cylindrical shapes.

17. A vision device, such as an endoscope comprising a tip housing according to any one of claims 11 to 16.
